(19) Europäisches Patentamt

European Patent Office

Office européen des brevets

(11) **EP 1 098 956 B1**

(12) **FASCICULE DE BREVET EUROPEEN**

(45) Date de publication et mention
de la délivrance du brevet:
**06.11.2002 Bulletin 2002/45**

(21) Numéro de dépôt: **99923661.5**

(22) Date de dépôt: **03.06.1999**

(51) Int Cl.⁷: **C12M 1/33**, C12N 1/06,
C12M 3/08

(86) Numéro de dépôt international:
**PCT/FR99/01309**

(87) Numéro de publication internationale:
**WO 00/005338 (03.02.2000 Gazette 2000/05)**

(54) **DISPOSITIF PERFECTIONNE ET PROCEDE DE LYSE DE MICRO-ORGANISMES**

VERFAHREN UND ANLAGE ZUR LYSE VON MICROORGANISMEN

IMPROVED DEVICE AND METHOD FOR LYSIS OF MICRO-ORGANISMS

(84) Etats contractants désignés:
**AT BE CH CY DE DK ES FI FR GB GR IE IT LI LU
MC NL PT SE**

(30) Priorité: **23.07.1998 FR 9809583**

(43) Date de publication de la demande:
**16.05.2001 Bulletin 2001/20**

(73) Titulaire: **BIO MERIEUX
69280 Marcy l'Etoile (FR)**

(72) Inventeurs:
• **BROYER, Patrick
F-69100 Villeurbanne (FR)**
• **CLEUZIAT, Philippe
F-69003 Lyon (FR)**
• **COLIN, Bruno
F-69280 Marcy l'Etoile (FR)**
• **PARIS, Cécile
F-69280 Marcy L'Etoile (FR)**
• **SANTORO, Lyse
F-69269 Charbonnières les Bains (FR)**

(74) Mandataire: **Guerre, Dominique et al
Cabinet Germain et Maureau,
12, rue Boileau,
BP 6153
69466 Lyon Cedex 06 (FR)**

(56) Documents cités:
**EP-A- 0 284 044          US-A- 5 643 767**

Remarques:
Le dossier contient des informations techniques
présentées postérieurement au dépôt de la
demande et ne figurant pas dans le présent
fascicule.

**Description**

La présente invention concerne un nouveau dispositif de lyse de micro-organisme permettant de libérer, par destruction totale ou partielle de la membrane, au moins un constituant biologique intracellulaire. Elle concerne également un procédé de lyse mettant en oeuvre un dispositif tel que défini ci-dessus.

*L'état de la technique, tel que la demande de brevet EP-A-0.284.044, décrit un procédé généralement utilisé pour lyser les cellules, qui consiste essentiellement à disposer, dans un contenant, un échantillon biologique en milieu liquide, comprenant le micro-organisme à lyser. Ensuite, on ajoute dans le contenant au moins un matériau sous forme de particules, relativement dur et substantiellement inerte par rapport aux composés biologiques. Enfin, on soumet le mélange, constitué de l'échantillon biologique et du matériau sous forme de particules à un mouvement de type vortex.*

Ce procédé utilisé présente certains inconvénients. Ainsi, il a été remarqué que le procédé de lyse couramment utilisé n'est pas suffisamment efficace, notamment la lyse cellulaire s'avère insuffisante en quantité et en qualité.

De plus, le procédé employé ne permet pas toujours de lyser des cellules réputées résistantes à la lyse, comme par exemple les cellules à Gram positif, telles que les Mycobactéries.

De même, la mise en oeuvre du procédé nécessite souvent l'ajout de réactifs supplémentaires, notamment des enzymes et/ou des détergents.

*La demanderesse a déposé une demande de brevet FR97/12164 en date du 23 septembre 1997 concernant un procédé de lyse de micro-organisme.*

*Ce procédé de lyse a pour objet de libérer au moins un composé biologique, contenu dans le micro-organisme, comprenant une étape de lyse, selon laquelle :*

- *on dispose dans un contenant un échantillon biologique en milieu liquide, comprenant le micro-organisme à lyser,*
- *on dispose dans ledit contenant au moins un matériau sous forme de particules, relativement dur et substantiellement inerte par rapport aux composés biologiques, et*
- *on soumet le mélange de l'échantillon biologique et du matériau sous forme dE particules à un mouvement de type vortex.*

  *Le procédé utilise un matériau sous forme de particules qui comprend des billes ayant un diamètre compris entre 90 et 150 /μm, et qui répond à l'équation suivante ;*

$$Ve = \alpha Vb,$$

*où Vb est le volume apparent des billes, Ve est le volume de l'échantillon liquide et α est un nombre compris entre 1,4 et 10, lorsque le contenant est de forme tubulaire, et est inférieur ou égal à 2, 1, lorsque ledit conterrant est de forme discoïde.*

Ces caractéristiques restent particulièrement intéressantes et le contenu de cette demande de brevet doit être considéré comme contenu dans la description de la présente invention.

*Le document US-A-5,643,767 propose un récipient permettant l'isolement de composants cellulaires, tels que des acides ribonucléiques (ARN) à partir de cellules présentes dans une solution liquide. Ce récipient contient pour cela deux types de billes, qui se différencient par leur diamètre. Le premier type de faible dimension est en verre ou en métal avec un diamètre compris entre 0,1 et 1 millimètre. Le second type de dimension importante est dans la même matière, en verre ou en métal, avec un diamètre compris entre 3 et 5 millimètres.*

L'inconvénient essentiel de ce dispositif réside dans le fait que le mouvement des billes est homogène que le vortex soit mécanique ou magnétique.

Pourtant, la demanderesse a maintenu, par des efforts soutenus et des travaux complémentaires, ces études dans le domaine de la lyse magnétique à mouvement de type vortex, et a réussi à déterminer des caractéristiques en permettant une agitation de type vortex dans un contenant, sans avoir à agiter mécaniquement le contenant, comme c'est le cas avec un vortex mécanique. Ainsi, en ajoutant des billes de sensiblement deux diamètres différents, il est possible d'accroître considérablement l'efficacité de ladite lyse, en améliorant la libération des constituants intracellulaires. Il est également possible, à efficacité de lyse identique, de diminuer la durée de cette lyse, ce qui peut s'avérer particulièrement intéressant dans le cas de tests de diagnostic qui doivent être rapidement réalisés.

A cet effet, la présente invention concerne un dispositif de lyse d'au moins une espèce de micro-organisme, pour libérer au moins un constituant biologique intracellulaire, qui comprend un contenant destiné à recevoir un échantillon biologique, en milieu liquide, qui contient le micro-organisme à lyser, dans ce contenant étant présent au moins un matériau sous forme de particules, relativement dur et substantiellement inerte par rapport aux composés biologiques de l'échantillon, caractérisé par le fait que le matériau sous forme de particules comprend au moins deux types de moyens de broyage de dimensions différentes :

- au moins un moyen magnétique de grande dimension, qui est asservi au mouvement d'un champ magnétique, et
- au moins un moyen de petite dimension, qui est mis en mouvement par le moyen de broyage de grande dimension.

Le matériau sous forme de particules comprend, comme moyen de broyage de grande dimension, au moins une bille de grand diamètre, et, comme moyen de broyage de petite dimension, au moins une bille de petit diamètre.

Le rapport existant entre les dimensions du moyen de broyage de petite dimension et les dimensions du moyen de broyage de grande dimension est compris entre 1/10 et 1/100, préférentiellement entre 1/30 et 1/60 et, plus précisément, ce rapport est de 1/40.

De plus, le rapport existant entre les dimensions du micro-organisme à lyser et les dimensions du moyen de broyage de petite dimension est compris entre 1/10 et 1/100, préférentiellement entre 1/30 et 1/60 et plus précisément est de 1/50.

Autre caractéristique, le rapport existant entre le volume total du moyen de broyage de petite dimension et le volume de l'échantillon biologique, contenant le micro-organisme à lyser, est compris entre 1/2 et 1/5, et est, préférentiellement, de 1/3.

Le nombre de moyen(s) de broyage de grande dimension est compris entre 1 et 10 et, préférentiellement, entre 1 et 4.

Dans un premier mode préférentiel de réalisation, le contenant est de forme tubulaire.

Dans un second mode préférentiel de réalisation, le contenant est de forme discoïde.

Quel que soit le mode de réalisation utilisé, chaque moyen de broyage magnétique de grande dimension est mû par le mouvement rotatif autour du tube d'au moins un aimant permanent.

De plus, chaque moyen de broyage magnétique de grande dimension est mû par le mouvement rotatif sous le tube d'au moins un aimant permanent.

Dans le cas où le contenant est de forme discoïde, chaque moyen de broyage magnétique de grande dimension coopère avec un chemin de roulement sensiblement circulaire, situé à la périphérie du contenant.

La présente invention concerne également un procédé de lyse d'au moins une espèce de micro-organisme d'un échantillon biologique, pour libérer au moins un constituant biologique intracellulaire, qui utilise, dans un contenant, au moins un matériau sous forme de particules, formant moyen de broyage, relativement dur et substantiellement inerte par rapport aux composés biologiques de l'échantillon, caractérisé en ce qu'il consiste à :

- disposer dans le contenant au moins un moyen de broyage magnétique de grande dimension, au moins un moyen de broyage non magnétique de petite dimension et un échantillon biologique qui contient le micro-organisme à lyser, le moyen de broyage de grande dimension étant de dimension supérieure au moyen de broyage de petite dimension, et le moyen de broyage de petite dimension étant mis en mouvement, directement ou indirectement, par le moyen de broyage de grande dimension.
- placer le moyen de broyage magnétique dans un champ magnétique mobile, afin d'engendrer dans le mélange un mouvement de vortex, et
- stopper l'effet du champ magnétique sur ledit moyen de broyage magnétique.

Dans un premier mode de fonctionnement, le champ magnétique est mobile en rotation autour du contenant à hauteur de l'échantillon biologique.

Dans un second mode de fonctionnement, le champ magnétique est mobile. en rotation sous le contenant à hauteur de l'échantillon biologique.

Quel que soit le mode de fonctionnement, le champ magnétique mobile en rotation applique un champ alternativement fort et. faible au moyen de broyage. magnétique situé dans l'échantillon biologique.

Les figures ci-jointes sont données à titre d'exemple explicatif et n'ont aucun caractère limitatif. Elles permettront de mieux comprendre l'invention.

La figure 1 représente une vue en perspective d'un dispositif de lyse, selon l'invention, dans un mode de réalisation de forme discoïde.

La figure 2 représente une vue en coupe selon A-A du dispositif présenté à la figure 1.

La figure 3 représente une vue en gros plan du détail B mis en évidence à la figure 2.

La figure 4 représente une vue latérale et schématique d'un dispositif de lyse, selon la présente invention, dans un mode de réalisation de forme tubulaire, mais auquel est associé un premier type d'appareillage magnétique, permettant le bon fonctionnement du dispositif.

La figure 5 représente une vue sensiblement identique à celle présentée en figure 4, où le dispositif est associé à un second type d'appareillage magnétique, permettant le bon fonctionnement du dispositif.

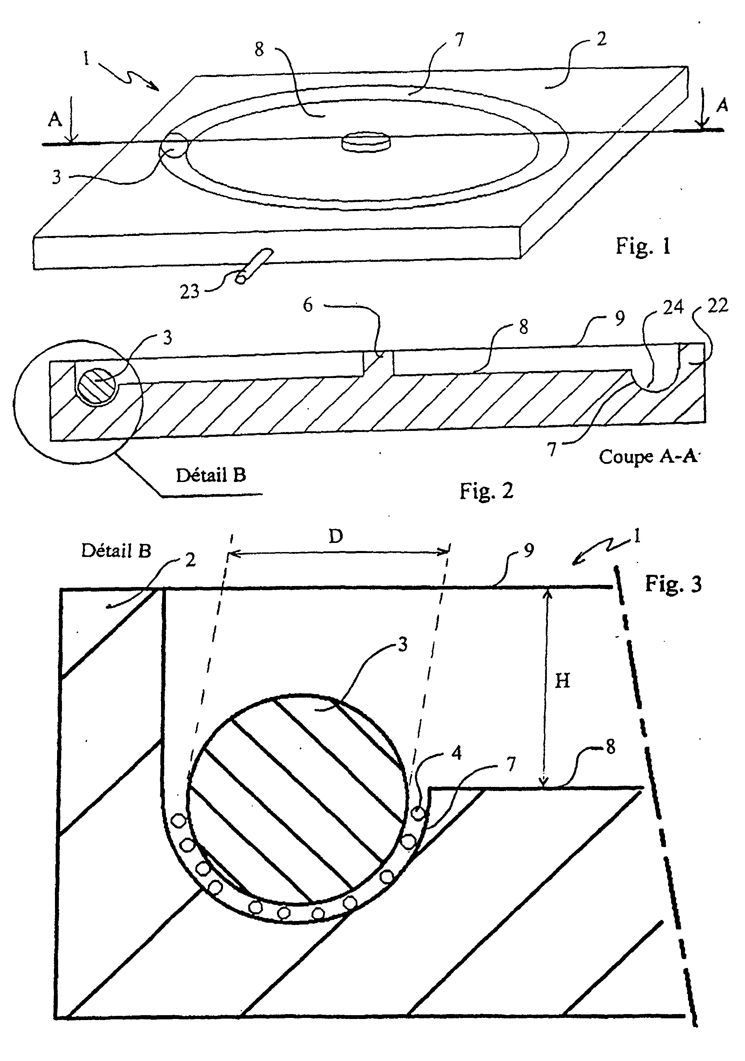

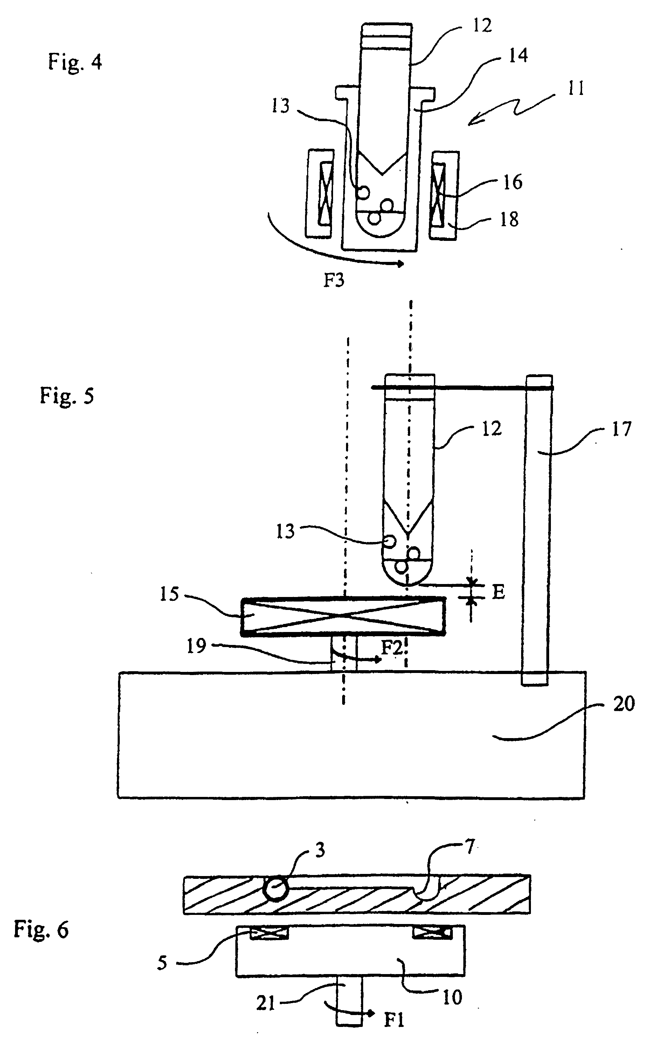

Enfin, la figure 6 représente une vue latérale et schématique du dispositif de lyse de forme discoïde, présenté aux figures 1 à 3, où ledit dispositif est associé à un troisième type d'appareillage magnétique, permettant le bon fonctionnement du dispositif de lyse.

La présente invention concerne plusieurs dispositifs de lyse bien représentés sur l'ensemble des figures 1 à 6.

Le premier dispositif de lyse 1 est représenté aux figures 1 à 3 et 6. Il est essentiellement constitué par un contenant 2, également appelé carte, de forme sensiblement parallélépipédique comportant, sur sa surface supérieure, une empreinte ou la lyse est réalisée.

Cette empreinte est essentiellement constituée par un chemin de roulement 7. Ce chemin 7 est situé à la périphérie de l'empreinte. Au centre de ladite empreinte est présent un téton 6, dont la surface supérieure, position qui est celle des figures 1 à 3, est au même niveau que la surface supérieure des bords latéraux 22. Située entre le téton central 6 et le chemin de roulement 7, est présente une plage intermédiaire 8. L'empreinte formée par le téton 6, le chemin de roulement 7 et la plage intermédiaire 8 est limitée par un moyen de cloisonnement également appelé film de cloisonnement 9.

Comme on le remarque bien sur la figure 3, le chemin de roulement 7 coopère avec deux moyens de broyage 3 et 4. Le premier moyen de broyage 3 est un moyen de broyage de grand diamètre alors que le moyen de broyage 4 est un moyen de broyage de petit diamètre. En fait, le moyen de broyage 3 est constitué par une bille magnétique 3, alors que le moyen de broyage 4 est constitué par une pluralité de billes de petit diamètre 4.

Toujours selon la figure 3, il est évident qu'il existe un rapport entre le diamètre D de la bille 3 et la hauteur H qui existe entre la plage intermédiaire 8 et le moyen de cloisonnement 9. Ainsi, pour éviter que la bille de grand diamètre 3 sorte du chemin de roulement 7, le diamètre D est supérieur à la hauteur H.

Il existe également un rapport entre les dimensions de la bille 3 et de chaque bille 4. Ainsi, la bille 3 est magnétique et est d'un diamètre de sensiblement 2 millimètres (mm) alors que les billes 4 sont en verre et sont d'un diamètre de cinquante micromètres ($\mu$m). Il existe donc un rapport entre chaque bille 4 et la bille 3, qui est d'un pour quarante.

De la même façon, il existe un rapport entre les billes de verre 3 de petit diamètre et les bactéries, qui sont en règle générale d'une taille d'un micron, le rapport existant entre bactérie et bille 4 est donc de un pour cinquante.

Il est tout à fait possible d'utiliser d'autres dimensions et donc d'autres rapports. Par exemple, des billes de 200 $\mu$m ont été utilisées avec succès, le rapport étant alors de un pour deux cents.

Bien entendu, la largeur du chemin de roulement 7 doit être légèrement supérieure au diamètre D de la bille de grand diamètre 3. Dans le cas présent, cette largeur du chemin 7 est de 2,2 mm.

De la même façon, il est important qu'il existe un rapport entre le volume total du moyen de broyage de petite dimension 4 et le volume de l'échantillon biologique contenant le micro-organisme à lyser. Ce rapport est en règle générale compris entre un pour deux et un pour cinq mais est préférentiellement de un pour trois.

Dans le mode de réalisation présenté sur les figures 1 à 3, il n'y a qu'une seule bille de grand diamètre 3 alors qu'il y a une multitude de billes de petit diamètre 4. La seule nécessité est bien sûr de respecter le rapport qui doit exister entre le volume de billes 4 et du volume de l'échantillon biologique. Bien entendu, l'échantillon biologique n'est pas représenté sur ces figures.

Selon la figure 1, un moyen d'introduction 23 de l'échantillon biologique à l'intérieur de l'espace délimité par le film de cloisonnement 9 et l'empreinte est représentée. Ce moyen d'introduction 23 débouche au sein de l'empreinte au niveau du chemin de roulement 7.

Le mouvement à l'intérieur du contenant 2 est engendré, comme cela est représenté à la figure 6, par l'intermédiaire d'aimants 5 présents au niveau d'un support 10. Ce support 10 est monté sur un axe de rotation 21. Cet axe de rotation 21 est associé à un moteur, non représenté sur cette figure mais qui est référencé 20 en figure 5, qui anime l'axe 21 d'un mouvement selon F1. Le mouvement de rotation F1 engendre un déplacement des aimants 5 qui peuvent être au nombre de deux comme cela est représenté sur la figure 6, mais dont le nombre n'est pas limitatif, il peut en avoir un ou trois, quatre ou plus. Quoiqu'il en soit, l'aimant 5 va entraîner en rotation sur elle-même mais également le long du chemin de roulement 7, la bille de grand diamètre 3 qui est en une matière magnétique. Le mouvement de la bille 3 va entraîner également le mouvement des billes de petit diamètre 4, qui sont en verre et qui ne sont donc bougées que par l'intermédiaire de cette bille 3 et du flux liquide engendré par le mouvement de ladite bille 3. Les diamètres plus petits des billes 4 vont augmenter les surfaces de contact entre les billes 4 et la paroi du chemin de roulement 7, d'une part, et entre lesdites billes de petit diamètre 4 et la bille de grand diamètre 3, d'autre part, permettant ainsi d'augmenter de manière significative le nombre de chocs et d'interactions (cisaillement) entre les billes de verre et les bactéries ou les levures, qui détruisent les membranes des micro-organismes et donc accentuent la libération des constituants biologiques intracellulaires qui est accrue.

Néanmoins, il est tout à fait possible d'utiliser le contenant 2 dans une autre position que celle représentée à la figure 6. Ainsi, il est possible de positionner l'orifice du moyen d'introduction 23, qui débouche dans le chemin de roulement 7, pour éviter que les petites billes 4 ne tombent dans ledit moyen 23 par simple gravité.

Selon un second mode de réalisation, représenté à la figure 4, le dispositif de lyse 11 utilise comme contenant un tube 12.

Ce contenant 12, comme le précédent référencé 2, est en polystyrène ou en polypropylène.

Deux appareillages sont décrits en figure 4 et en figure 5 permettant d'utiliser un tel tube 12.

Selon la figure 4, la lyse s'effectue dans le tube 12 par l'intermédiaire de moyens de broyage de grande dimension 13, qui sont associés bien entendu à des moyens de broyage de petite dimension, dans le même rapport établi pour le premier mode de réalisation représenté aux figures 1 à 3, mais qui ne sont pas représentés sur cette figure 4. Néanmoins, il sera assez évident de comprendre que le fonctionnement sera identique à l'exception du fait que le chemin de roulement est ici remplacé par les bords latéraux du tube 12. Ce tube 12 est situé dans un support 14 qui permet de bien positionner le tube 12 par rapport à des moyens. Ces moyens permettent de faire subir un champ magnétique aux billes magnétiques de grand diamètre 13 situées dans le contenant 12. Dans ce cas, on note la présence d'aimants permanents 16, situés autour du contenant 12, eux-mêmes solidarisés les uns aux autres par l'intermédiaire d'un support 18 en forme d'anneau représenté partiellement sur cette figure 4. L'ensemble support 18 et aimants 16 est animé d'un mouvement de rotation autour du tube 12 et de son support 14 selon F3, et entraîne ainsi le mouvement des billes de grand diamètre 13. Ces billes 13 sont ici au nombre de trois. Des tests ont également été réalisés avec cinq, dix ou quinze billes.

Selon la figure 5, le contenant 12 comporte également des billes de grand diamètre 13 au nombre de trois. Le support est ici constitué par une potence 17 qui maintient la partie supérieure du tube 12. En fait, l'aimant 15 est ici présent en position inférieure au tube 12, de sorte que le mouvement de rotation engendré au niveau du moteur 20 selon F2, est transmis à l'aimant 15 par l'intermédiaire de l'axe 19, ce qui va engendrer un mouvement au niveau des billes 13. Selon cette figure, les billes 13 sont soumises alternativement à un gradient de champ fort puis faible, lorsque l'aimant permanent s'éloigne ou se rapproche du tube 12, ce qui anime lesdites billes 13 d'un mouvement créant les forces de cisaillement dues aux billes de petit diamètre, non représentées également sur cette figure. Il convient de noter que pour un effet optimum de cette configuration, il est nécessaire que l'écart E entre le fond du tube 12 et l'aimant 15 soit le plus faible possible. Un écart E de 0,5 à 4 mm est utilisable, et préférentiellement de 1 mm. On remarque sur les figures 4 et 5 que l'échantillon biologique présent dans le tube 12 est animé d'un mouvement de vortex de sorte qu'il se crée une forme d'entonnoir au niveau de la partie supérieure libre du liquide. Concernant la figure 6, on remarque sur cette figure qu'il n'y a pas de téton 6 en partie centrale du contenant 2. En fait, ce téton n'est absolument pas obligatoire, car il n'a pas de rôle dans la fonction de lyse du système. Par contre, il a un rôle de soutien du film transparent 9, en polypropylène, qui délimite l'espace de lyse.

Les exemples qui vont suivre montrent la fonctionnalité des différents modes de réalisation du dispositif de lyse, ainsi que son efficacité par rapport au système classique de lyse mécanique.

Le mode de réalisation des figures 1 à 3 et 6 sera référencé, par la suite, carte discoïde, le mode de réalisation de la figure 4 sera, pour sa part, référencé vortex latéral, et le mode de réalisation de la figure 5 sera, quant à lui, référencé vortex longitudinal.

EXEMPLES

1 - Vortex magnétique en format carte (discoïde):

Ce mode de réalisation correspond sensiblement aux figures à à 3 et 6.

A - Mode opératoire :

Le protocole de lyse a été réalisé dans le contenant 2, format carte, sur deux types de micro-organismes mises en culture la veille, d'une part, des bactéries *Staphylococcus epidermidis* et, d'autre part, des levures *Candida krusei.*

La carte 2 utilisée a les caractéristiques suivantes. Les cotés sont d'une longueur de 4 centimètres (cm) et sa hauteur est de 0,4 cm. Elle est munie d'un puits non débouchant de hauteur d'environ 0,37 cm du fond sur chemin de roulement ou sillon 7 au bord de la face supérieure et de diamètre externe égal à 3 cm. Le diamètre du sillon 7 dans lequel évolue la bille de fer 3 est de 0,22 cm. La ou les billes de fer 3 sont de diamètre plus faible d'environ 0,19 cm afin d'avoir une mobilité importante. Dans les essais décrits, une seule bille de fer 3 a été utilisée.

Le puits est fermé par un film 9 autocollant transparent et rempli de billes de verre 4 de 100 micromètres (µm) pour la lyse des bactéries *S. epidermidis* et de 400 à 600 µm pour la lyse des levures *C. krusei.* La bille de fer 3 permettant d'engendrer l'agitation des billes de verre 4 est positionnée préalablement dans le sillon 7 de la carte 2. Ladite carte 2 est remplie avec la suspension de micro-organismes à lyser et enfin positionnée de manière horizontale sur le système d'aimants 5 tournants.

Le protocole de lyse a été réalisé pendant une durée de 5 minutes (mn) à vitesse maximale, soit environ 2800 tours par minute (tr/mn). Dans chaque cas, une bille de fer 3 et 600 µl de volume échantillon (*S. epidermidis* ou *C. Krusei*) ont été utilisés. Le volume apparent des billes de verre 4 a varié de 30 à 180 µl. Des cartes 2 avec et sans plot ou téton central 6 (permettant un positionnement plus aisé du film autocollant 9) ont été indifféremment utilisées.

B - Résultats :

La mesure de D.O. n'étant pas suffisamment fiable pour évaluer le pourcentage de lyse. La propreté des billes de lyse 4 (100 et 500 μm) étant de mauvaise qualité, un relargage important de particules issues desdites billes 4 semble se produire durant la lyse. Ce relargage modifie de manière significative la mesure de D.O. et biaise donc le résultat de lyse bactérienne. Dans un premier temps, les mesures de D.O. n'ont donc pas été utilisées mais ne sont cependant pas nécessaires pour démontrer l'efficacité des procédés de vortex magnétiques décrits.

Après l'étape de vortex magnétique, le lysat a été récupéré puis analysé sur gel d'agarose à 0,8 %. Le gel permet d'évaluer la quantité d'acides nucléiques libérée par la lyse (intensité de la bande) ainsi que leur état de conservation (bande très étroite et bien délimitée ou tache de grande longueur). Une photographie du gel montre dans sa partie supérieure, la migration des acides nucléiques de bactéries *S. epidermidis* libérée et dans sa partie inférieure, la migration des acides nucléiques de levures libérée.

Dans chaque cas, un témoin de migration, sur suspension initiale non lysée, a été déposé dans le premier puits. On vérifie sur le gel que dans les deux cas, aucune bande d'acide nucléique n'apparaît avant la lyse. Les résultats de lyse sont comparés également au résultat de la méthode de référence, c'est-à-dire la lyse par vortex mécanique, suivant les conditions expérimentales décrites dans le mode opératoire.

Pour les lysats de bactéries *S. epidermidis,* on constate que bien que l'intensité des bandes est faible vis-à-vis du témoin vortex mécanique, les bandes d'ADN et les deux bandes d'ARN sont visibles et plus ou moins intenses en fonction des conditions expérimentales.

Certains des essais, référencés 2, 4, 5, 6, 7 et 8 ont été obtenus avec 30 μl de billes 4 NIAI, d'un diamètre de 100 μm, dans 600 μl de suspension bactérienne. On constate que les bandes sont visibles mais de très faible intensité ce qui signifie que peu d'acides nucléiques ont été libérés.

Un essai, référencé 1, a été réalisé avec 180 μl. La bande d'acides nucléiques est également peu visible. Ceci s'explique par la trop grande quantité de billes 4 vis-à-vis de la dimension du sillon 7 dans lequel tourne la bille de fer 3. La vitesse de rotation de la bille 3 étant faible, l'agitation des billes 4 est peu efficace et par conséquent la libération des acides nucléiques peu importante.

Un autre essai, référencé 3, a été réalisé avec 90 μl de billes 4 dans 600 μl de suspension. On voit clairement sur le gel que l'intensité des bandes est nettement supérieure aux précédents essais. Le gel montre également que les acides nucléiques sont dans le même état de conservation que ceux libérés avec le vortex mécanique.

En conclusion, l'efficacité de lyse est nettement dépendante du ratio de billes 3 et 4 vis-à-vis du volume d'échantillon, et dépend également du ratio entre le volume d'échantillon et le volume total du puits discoïde 24.

Dans ces essais, la quantité de billes 3 et 4 optimisée se situe vraisemblablement, pour le contenant discoïde 2 testé, entre 90 et 180 μl. Le choix des rapports corrects entre quantité de billes, quantité d'échantillon et volume du puits permet d'améliorer significativement l'efficacité de lyse et d'obtenir une performance identique à celle du vortex mécanique, mais sans en avoir les inconvénients, tels que le bruit, les vibrations.

Pour les lysats de levures *C. krusei,* comme pour les suspensions bactériennes, on constate que l'intensité des bandes d'acides nucléiques, c'est-à-dire la quantité de matériel libéré, dépend des conditions expérimentales.

Les essais, référencés 6 et 7, ont été réalisés avec 30 μl de billes 4 de 500 μm dans 600 μl de suspension de levures. On constate que les bandes sont visibles, mais de très faible intensité ce qui signifie que peu d'acides nucléiques ont été libérés. La quantité de billes 4 est donc trop faible vis-à-vis du volume d'échantillon à lyser.

Les essais, référencés 1 et 2, ont été réalisés avec 60 μl de billes 4, toujours dans 600 μl de suspension. Les bandes d'acides nucléiques sont un peu plus visible mais restent peu intenses vis-a-vis des témoins vortex mécaniques. Lors de ces essais, des blocages importants lors de la rotation de la bille de fer 3 ont été notés, ainsi qu'une stagnation importante au niveau du plot central 6 d'une partie de la suspension à lyser. Ceci explique la différence avec les essais référencés 3 et 4 pour lesquels les bandes ADN/ARN sont nettement visibles et plus intenses que pour les autres essais.

On notera que, comme pour les suspensions bactériennes, les acides nucléiques sont parfaitement conservés.

Les mêmes conclusions que pour les essais sur les bactéries sont valables. Il est nécessaire d'optimiser la quantité de billes de verre 4 vis-à-vis du volume d'échantillon ainsi que le volume du puits 24. La vitesse de rotation de la bille 3 et la durée de vortex sont deux facteurs également très importants influençant directement la quantité d'acides nucléiques libérés.

2 - Vortex magnétique en format tubulaire (latéral):

Ce mode de réalisation correspond sensiblement à la figure 4.

A - Mode opératoire :

Le protocole de lyse a été réalisé dans le contenant 12 format tube type Falcon à fond en forme de U (diamètre x hauteur = 12 x 75 mm) sur bactéries *S. epidermidis* mises en culture la veille.

Les tubes 12 ont été remplis d'un volume de 600 µl de suspension bactérienne et d'un volume apparent de 180 µl de billes 4 VIAI de diamètre 100 µm. Différents réglages ont été testés pour le nombre de billes de fer 3, entraînant l'agitation de la suspension, pour le nombre de billes de verre 4 d'un diamètre de 3 mm, pour la durée de vortex et pour la vitesse de rotation .

Les conditions expérimentales testées sont résumées dans le tableau 1 ci-dessous, où la D.O. de la suspension initiale est de 0,672. A ce propos, le calcul du pourcentage de lyse est calculé en retirant la valeur de D.O. parasite à la valeur mesurée. Ces valeurs sont toutefois uniquement indicatives et ne doivent pas être considérées comme un critère permettant de déterminer le réglage optimal pour les paramètres étudiés.

Tableau 1 :

| Etude de l'efficacité d'un vortex magnétique en format tubulaire subissant une aimantation latérale | | | | | | |
|---|---|---|---|---|---|---|
| N° | DO | % lyse | Billes Fer | Billes Verre | Durée (mn) | Tension (V) |
| 1 | 0,176 | 0 | 10 | 6 | 2 | Vortex Mécanique BCC stérile |
| 2 | 0,338 | 75,9 | 10 | 6 | 2 | Vortex Mécanique *S. epidermidis* |
| 3 | 0,171 | 0 | 10 | 6 | 5 | 6,5 |
| 4 | 0,558 | 42,4 | 5 | 0 | 5 | 5 |
| 5 | 0,577 | 39,6 | 5 | 0 | 5 | 5,5 |
| 6 | 0,613 | 34,2 | 5 | 0 | 5 | 6 |
| 7 | 0,627 | 32,1 | 5 | 0 | 5 | 6,5 |
| 8 | 0,698 | 21,6 | 10 | 0 | 5 | 5 |
| 9 | 0,542 | 44,8 | 10 | 0 | 5 | 5,5 |
| 10 | 0,614 | 34,1 | 10 | 0 | 5 | 6 |
| 11 | 0,639 | 30,3 | 10 | 0 | 5 | 6,5 |
| 12 | 0,562 | 41,8 | 15 | 0 | 5 | 5 |
| 13 | 0,662 | 26,9 | 15 | 0 | 5 | 5,5 |
| 14 | 0,646 | 29,3 | 15 | 0 | 5 | 6 |
| 15 | 0,643 | 29,8 | 15 | 0 | 5 | 6,5 |
| 16 | 0,696 | 21,9 | 5 | 3 | 5 | 5 |
| 17 | 0,598 | 36,5 | 5 | 3 | 5 | 5,5 |
| 18 | 0,661 | 27,1 | 5 | 3 | 5 | 6 |
| 19 | 0,63 | 31,7 | 5 | 3 | 5 | 6,5 |
| 20 | 0,632 | 31,4 | 10 | 3 | 5 | 5 |
| 21 | 0,608 | 35 | 10 | 3 | 5 | 5,5 |
| 22 | 0,652 | 28,4 | 10 | 3 | 5 | 6 |
| 23 | 0,655 | 28 | 10 | 3 | 5 | 6,5 |
| 24 | 0,672 | 25,4 | 15 | 3 | 5 | 5 |
| 25 | 0,599 | 36,3 | 15 | 3 | 5 | 5,5 |
| 26 | 0,607 | 35,1 | 15 | 3 | 5 | 6 |
| 27 | 0,661 | 27,1 | 15 | 3 | 5 | 6,5 |
| 28 | 0,58 | 39,1 | 15 | 3 | 10 | 6,5 |

Tableau 1 :   (suite)

| Etude de l'efficacité d'un vortex magnétique en format tubulaire subissant une aimantation latérale | | | | | | |
|---|---|---|---|---|---|---|
| N° | DO | % lyse | Billes Fer | Billes Verre | Durée (mn) | Tension (V) |
| 29 | 0,489 | 52,7 | 15 | 3 | 10 | 7,5 |
| 30 | 0,607 | 35,1 | 15 | 3 | 5 | 6,5 |
| 31 | 0,522 | 47,8 | 15 | 3 | 10 | 6,5 |
| 32 | 0,636 | 30,8 | 15 | 3 | 5 | 13 |
| 33 | 0,594 | 37 | 15 | 3 | 10 | 13 |

B - Résultats :

Comme pour les essais avec le vortex magnétique en carte (discoïde), seule l'évaluation sur gel d'agarose à 0,8 % a été utilisée pour démontrer l'efficacité du procédé, étant donné le biais induit sur les mesures de D.O. par les impuretés libérées par les billes de verre 4.

La photographie du gel montre tout d'abord que, quelque soit les conditions expérimentales, une libération d'acides nucléiques a été engendrée pour les essais pratiqués avec le vortex latéral, sous les références 1 à 32.

La bande d'ADN est visible ainsi que les deux bandes ARN. Comparativement au vortex mécanique, la qualité de conservation des acides nucléiques est identique.

Ces essais nous permettent de définir la zone de réglage (temps, nombre de billes de grande dimension 13 ou de petite dimension, non représentées sur la figure 4, hauteur des aimants 16, vitesse de rotation) dans laquelle le vortex magnétique latéral fonctionne de manière satisfaisante. On constate que les essais 1 à 7, 10, 17 à 26, 29 et 30 sont très satisfaisants du point de vue quantité et qualité des acides nucléiques libérés comparativement au vortex mécanique.

Le réglage optimal du vortex magnétique latéral semble se situer autour du point suivant pour 600 µl d'échantillon :

- 180 µl de billes de verre 4 VIAI (100 µm),
- cinq billes 3 de fer (diamètre 2 mm),
- 10 mn (plus la durée est importante plus la lyse est importante),
- entre 2800 et 3700 tr/mn de vitesse de rotation des aimants autour du tube,
- deux aimants NdFeB, référence NE 129-2 provenant par exemple de Binder Magnetic,
- environ 4 mm pour la distance la plus proche entre aimant 16 et tube 12, et
- surélévation du tube 12 vis-à-vis du fond du fourreau de tube.

Cette surélévation définie la position relative des aimants 16 par rapport au tube 12.

Ces essais démontrent l'efficacité du vortex magnétique latéral. La durée de lyse intervient directement sur la quantité d'acides nucléiques libérés jusqu'au plateau de saturation situé aux alentours 90%.

3 - Vortex magnétique en format tubulaire (longitudinal) :

Ce mode de réalisation correspond sensiblement à la figure 5.

A - Mode opératoire :

Le protocole de lyse a été réalisé dans un contenant 12 format tube type Falcon à fond en forme de U (diamètre x hauteur = 12 x 75 mm) sur bactéries *S. epidermidis* mises en culture la veille.

Les tubes 12 ont été remplis d'un volume de 600 µl de suspension bactérienne et d'un volume apparent de 180 µl de billes, non représentées sur cette figure 5, VIAI de diamètre 100 µm puis fermés par un bouchon. Le tube 12 est fixé au support mécanique ou potence 17, comportant un bras, par la partie supérieure de son corps, et placé de manière asymétrique par rapport à l'axe de rotation 19 de l'aimant 15. La distance entre l'aimant 15 et le fond externe du tube est fixée à environ 2 mm. L'aimant permanent 15, fixé sur le rotor du système mécanique est de type NdFeB, référence NE 1 030 de chez Binder Magnetic.

Des tests ont été réalisés pour différents paramètres, tels que le nombre de billes de fer 13 (5, 10 ou 15), la durée de vortex (5 ou 10 mn) et la vitesse de rotation (2800 tr/mn, 1860 tr/mn et 930 tr/mn). Le maintien ou non du tube afin d'empêcher sa vibration a également été testé.

Les conditions expérimentales testées sont résumées dans le tableau 2 ci-dessous. La D.O. de la suspension initiale est de 0,672.

Tableau 2 :

| Etude de l'efficacité d'un vortex magnétique en format tubulaire subissant une aimantation longitudinale | | | | | |
|---|---|---|---|---|---|
| **N°** | **DO (550 nm)** | **Billes Fer** | **Billes Verre** | **Durée (mn)** | **Vitesse** |
| S.I. (Suspension Initiale) | 0,672 | | | | |
| Témoin | 0,398 | | | | |
| Tmoysteril 10* | 0,365 | 5 à 15 | 0 | 10 | 9 |
| Tmoysteril 5* | 0,3 | 5 à 10 | 0 | 5 | 9 |
| 9 | 0,313 | 10 | 0 | 10 | 9 |
| 9' | 0,278 | 10 | 0 | 10 | 9 |
| 10 | 0,245 | 15 | 0 | 10 | 9 |
| 10' | 0,199 | 15 | 0 | 10 | 9 |
| 21 | 0,126 | 10 | 0 | 10 | 9 |
| 22 | 0,2 | 15 | 0 | 10 | 9 |
| 11 | 0,217 | 10 | 0 | 10 | 6 |
| 11' | 0,099 | 10 | 0 | 10 | 6 |
| 12 | 0,224 | 15 | 0 | 10 | 6 |
| 12' | 0,237 | 15 | 0 | 10 | 6 |
| 13 | 0,13 | 5 | 0 | 10 | 9 |
| 14 | 0,155 | 10 | 0 | 10 | 9 |
| 15 | 0,305 | 5 | 0 | 5 | 9 |
| 16 | 0,292 | 10 | 0 | 5 | 9 |
| 17 | 0,242 | 5 | 0 | 10 | 6 |
| 18 | 0,091 | 10 | 0 | 10 | 6 |
| 19 | 0,261 | 5 | 0 | 5 | 6 |
| 20 | 0,334 | 10 | 0 | 5 | 6 |
| 30 | 0,568 | 5 | 0 | 10 | 3 |
| 31 | 0,57 | 10 | 0 | 10 | 3 |
| 32 | 0,667 | 15 | 0 | 10 | 3 |
| 36 | 0,655 | 5 | 0 | 10 | 3 |
| 37 | 0,62 | 10 | 0 | 10 | 3 |
| 33 | 0,583 | 5 | 0 | 5 | 3 |
| 34 | 0,466 | 10 | 0 | 5 | 3 |

Tmoystéril 10* : Il s'agit d'un témoin à base d'un tampon BCC stérile (c'est-à-dire sans micro-organisme) traité par un vortex magnétique, afin de déterminer l'augmentation du bruit de fond de la D.O. dû au relargage des impuretés. La D.O. devrait être d'environ 0 s'il n'y avait pas des saletés. Il s'agit d'une moyenne sur 3 tubes pendant 10 mn.

T moy stérile 5* : Il s'agit d'un témoin à base d'un tampon BCC stérile (c'est-à-dire sans micro-organisme) traité par un vortex magnétique, afin de déterminer l'augmentation du bruit de fond de la D.O. dû au relargage des impuretés. La D.O. devrait être d'environ 0 s'il n'y avait pas des saletés. Il s'agit d'une moyenne sur 3 tubes pendant 5 mn.

Tableau 2 :   (suite)

| Etude de l'efficacité d'un vortex magnétique en format tubulaire subissant une aimantation longitudinale | | | | | |
|---|---|---|---|---|---|
| N° | DO (550 nm) | Billes Fer | Billes Verre | Durée (mn) | Vitesse |
| 35 | 0,82 | 15 | 0 | 5 | 3 |
| 38 | 0,638 | 5 | 0 | 5 | 3 |
| 39 | 0,627 | 10 | 0 | 5 | 3 |
| 40 | 0,764 | 5 | 0 | 5 | 3 |
| 41 | 0,751 | 5 | 0 | 5 | 3 |

B - Résultats :

L'évaluation de la lyse bactérienne a été effectuée sur gel d'agarose à 0,8 % pour démontrer l'efficacité du procédé étant donné le biais induit sur les mesures de D.O. par les impuretés libérées par les billes de verre.

Une photographie du gel montre tout d'abord que, dans la majorité des conditions expérimentales, une libération d'acides nucléiques a été engendrée pour les essais pratiqués avec le vortex longitudinal, référencés 9 à 41 du tableau 2.

La bande d'ADN est visible ainsi que les deux bandes d'ARN 16 et 23S. Comparativement au vortex mécanique, référencé T pour témoin, la qualité de conservation des acides nucléiques est identique. Dans la majorité des cas expérimentaux, l'intensité des bandes d'ADN/ARN est égale ou supérieure au vortex mécanique. A condition de trouver le réglage optimal pour les paramètres d'influence, l'efficacité de ce vortex magnétique est donc supérieure ou égale au vortex mécanique pris comme référence.

La zone de réglage (temps, nombre de billes, hauteur des aimants, vitesse de rotation) dans laquelle le vortex magnétique latéral fonctionne de manière satisfaisante est donc déterminée. On constate que les essais 9, 9' et 12 à 35 sont très satisfaisants du point de vue quantité et qualité des acides nucléiques libérés comparativement au vortex mécanique.

Le réglage optimal du vortex magnétique longitudinal se situe autour du point suivant pour 600 μl d'échantillon :

- 180 μl de billes de verre VIAI (100 μm),
- cinq à dix billes de fer 13 d'un diamètre de 2 mm,
- 10 mn pour la durée de lyse,
- entre 930 et 2800 tr/mn pour la vitesse de rotation des aimants,
- un aimant NdFeB, référence NE 1 030 de chez Binder Magnetic, et
- 2 mm environ pour la distance entre aimant 15 - tube 12.

Il convient de noter de caractéristiques intéressantes, d'une part, plus la durée de lyse, plus elle est efficace et, d'autre part, le tube 12 est laissé libre en vibration, afin d'entraîner une plus grande agitation des billes de fer 13 et de verre.

L'efficacité du vortex magnétique longitudinal est donc démontrée, même si le réglage optimal n'est pas correctement défini. La durée de lyse intervient directement sur la quantité d'acides nucléiques libérés jusqu'au plateau de saturation vers 90%.

**REFERENCES**

1. Dispositif de lyse
2. Contenant discoïde
3. Moyen de broyage de grande dimension
4. Moyen de broyage de petit dimension
5. Aimant permanent sous le contenant 2
6. Téton central
7. Chemin de roulement
8. Plage intermédiaire

9. Moyen de cloisonnement ou film
10. Support du ou des aimants 5
11. Dispositif de lyse
12. Contenant tubulaire
13. Moyen de broyage de grande dimension
14. Support du contenant 12
15. Aimant permanent sous le contenant 12
16. Aimant permanent autour du contenant 12
17. Potence du contenant 12
18. Support d'aimant(s) 16
19. Axe de rotation de l'aimant 15
20. Moteur transmettant son énergie par l'axe 19
21. Axe de rotation du support 10 et des aimants 5
22. Bords latéraux du contenant 2
23. Moyen d'introduction de l'échantillon biologique
24. Puits discoïde
F1. Mouvement de rotation du support 10 des aimants 5
F2. Mouvement de rotation de l'aimant 15
F3. Mouvement de rotation du support 18 des aimants 16
D. Diamètre d'un moyen de broyage de grande dimension 3
E. Ecartement entre le fond du contenant 12 et l'aimant 15
H. Hauteur entre la plage intermédiaire 8 et le film de cloisonnement 9

**Revendications**

**1.** Dispositif de lyse (1 ou 11) d'au moins une espèce de micro-organisme, pour libérer au moins un constituant biologique intracellulaire, qui comprend un contenant (2 ou 12) destiné à recevoir un échantillon biologique, en milieu liquide, qui contient le micro-organisme à lyser, dans ce contenant étant présent au moins un matériau sous forme de particules, relativement dur et substantiellement inerte par rapport aux composés biologiques de l'échantillon, **<u>caractérisé par le fait que</u>** le matériau sous forme de particules comprend au moins deux types de moyen de broyage de dimensions différentes :

- au moins un moyen magnétique de grande dimension (3 ou 13), qui est asservi au mouvement d'un champ magnétique, et
- au moins un moyen de petite dimension (4), qui est mis en mouvement par le moyen de broyage de grande dimension (3 ou 13).

**2.** Dispositif, selon la revendication 1, **<u>caractérisé par le fait que</u>** le matériau sous forme de particules comprend, comme moyen de broyage de grande dimension (3 ou 13), au moins une bille de grand diamètre (3 ou 13), et, comme moyen de broyage de petite dimension (4), au moins une bille de petit diamètre (4).

**3.** Dispositif, selon l'une quelconque des revendications 1 ou 2, **<u>caractérisé par le fait que</u>** le rapport existant entre les dimensions du moyen de broyage de petite dimension (4) et les dimensions du moyen de broyage de grande dimension (3 ou 13) est compris entre 1/10 et 1/100, préférentiellement entre 1/30 et 1/60 et, plus précisément, ce rapport est de 1/40.

**4.** Dispositif, selon l'une quelconque des revendications 1 à 3, **<u>caractérisé par le fait que</u>** le rapport existant entre les dimensions du micro-organisme à lyser et les dimensions du moyen de broyage de petite dimension (4) est compris entre 1/10 et 1/100, préférentiellement entre 1/30 et 1/60 et plus précisément est de 1/50.

**5.** Dispositif, selon l'une quelconque des revendications 1 à 4, **<u>caractérisé par le fait que</u>** le rapport existant entre le volume total du moyen de broyage de petite dimension (4) et le volume de l'échantillon biologique, contenant le micro-organisme à lyser, est compris entre 1/2 et 1/5, et est, préférentiellement, de 1/3.

**6.** Dispositif, selon l'une quelconque des revendications 1 à 5, **<u>caractérisé par le fait que</u>** le nombre de moyen(s) de broyage de grande dimension (3 ou 13) est compris entre 1 et 10 et, préférentiellement, entre 1 et 4.

**7.** Dispositif, selon l'une quelconque des revendications 1 à 6, **caractérisé par le fait que** le contenant (12) est de forme tubulaire.

**8.** Dispositif, selon l'une quelconque des revendications 1 à 6, **caractérisé par le fait que** le contenant (12) est de forme discoïde.

**9.** Dispositif, selon la revendication 7 ou 8, **caractérisé par le fait que** chaque moyen de broyage magnétique de grande dimension (3 ou 13) est mû par le mouvement rotatif autour du contenant (2 ou 12) d'au moins un aimant permanent (16).

**10.** Dispositif, selon la revendication 7 ou 8, **caractérisé par le fait que** chaque moyen de broyage magnétique de grande dimension (3 ou 13) est mû par le mouvement rotatif sous le contenant (2 ou 12) d'au moins un aimant permanent (5 ou 15).

**11.** Dispositif, selon la revendication 8 et selon l'une quelconque des revendications 9 ou 10, **caractérisé par le fait que** chaque moyen de broyage magnétique de grande dimension (3) coopère avec un chemin de roulement sensiblement circulaire (7), situé à la périphérie du contenant (2).

**12.** Procédé de lyse d'au moins une espèce de micro-organisme d'un échantillon biologique, pour libérer au moins un constituant biologique intracellulaire, qui utilise, dans un contenant, au moins un matériau sous forme de particules, formant moyen de dans un contenant, au moins un matériau sous forme de particules, formant moyen de broyage, relativement dur et substantiellement inerte par rapport aux composés biologiques de l'échantillon, **caractérisé en ce qu'**il consiste à :

- disposer dans le contenant au moins un moyen de broyage magnétique de grande dimension, au moins un moyen de broyage non magnétique de petite dimension et un échantillon biologique qui contient le micro-organisme à lyser, le moyen de broyage de grande dimension étant de dimension au moyen de broyage de petite dimension, et le moyen de broyage de petite dimension étant mis en mouvement, directement ou indirectement, par le moyen de broyage de grande dimension.
- placer le moyen de broyage magnétique dans un champ magnétique mobile, afin d'engendrer dans le mélange un mouvement de vortex, et
- stopper l'effet du champ magnétique sur ledit moyen de brayage magnétique.

**13.** Procédé, selon la revendication 12, **caractérisé en ce que** le champ magnétique est mobile en rotation autour du contenant à hauteur de l'échantillon biologique.

**14.** Procédé, selon la revendication 12, **caractérisé en ce que** le champ magnétique est mobile en rotation sous le contenant à hauteur dé l'échantillon biologique.

**15.** Procédé, selon l'une quelconque des revendications 13 ou 14, **caractérisé en ce que** le champ magnétique mobile en rotation applique un champ alternativement fort et faible au moyen de broyage magnétique situé dans l'échantillon biologique.

**Patentansprüche**

**1.** Vorrichtung zum Lysieren (1 oder 11) von zumindest einem Typ an Mikroorganismen, um zumindest einen intrazellulären biologischen Bestandteil freizusetzen, mit einem Behältnis (2 oder 12), das dazu vorgesehen ist, in flüssigem Medium eine biologische Probe, die den zu lysierenden Mikroorganismus enthält, aufzunehmen, wobei in dem Behältnis zumindest ein Material in Form von relativ harten und gegenüber den biologischen Bestandteilen der Probe im wesentlichen inerten Teilchen enthalten ist, **dadurch gekennzeichnet, daß** das Material in Form von Teilchen zumindest zwei Arten an Mitteln zum Zerkleinern mit unterschiedlichem Ausmaß aufweist, nämlich

- zumindest ein magnetisches Mittel mit großem Ausmaß (3 oder 13), das durch die Bewegung eines Magnetfeldes gesteuert wird, und
- zumindest ein Mittel kleinen Ausmaßes (4), das durch die Mittel zum Zerkleinern mit großem Ausmaß (3 oder 13) bewegt wird.

**2.** Vorrichtung nach Anspruch 1, **dadurch gekennzeichnet, daß** das Material in Form von Teilchen als Mittel zum Zerkleinern mit großem Ausmaß (3 oder 13) zumindest eine Kugel mit großem Durchmesser (3 oder 13) aufweist, und daß die Mittel zum Zerkleinern mit kleinem Ausmaß (4) zumindest eine Kugel mit kleinem Durchmesser (4) aufweisen.

**3.** Vorrichtung nach Anspruch 1 oder 2, **dadurch gekennzeichnet, daß** das Verhältnis der Ausmaße der Mittel zum Zerkleinern mit kleinem Ausmaß (4) und den Ausmaßen der Mittel zum Zerkleinern mit großem Ausmaß (3 oder 13) zwischen 1/10 und 1/100, vorzugsweise zwischen 1/30 und 1/60 liegt, und höchst genau ausgedrückt liegt dieses Verhältnis bei 1/40.

**4.** Vorrichtung nach einem der Ansprüche 1 bis 3, **dadurch gekennzeichnet, daß** das Verhältnis zwischen den Ausmaßen des zu lysierenden Mikroorganismus und den Ausmaßen der Mittel zum Zerkleinern mit kleinem Ausmaß (4) zwischen 1/10 und 1/100, vorzugsweise zwischen 1/30 und 1/60, liegt, und höchst genau ausgedrückt ist es 1/50.

**5.** Vorrichtung nach einem der Ansprüche 1 bis 4, **dadurch gekennzeichnet, daß** das Verhältnis zwischen dem Gesamtvolumen der Mittel zum Zerkleinern mit geringem Ausmaß (4) und dem Volumen der biologischen Probe, die den zu lysierenden Mikroorganismus enthält, zwischen 1/2 und 1/5 liegt, und vorzugsweise 1/3 ist.

**6.** Vorrichtung nach einem der Ansprüche 1 bis 5, **dadurch gekennzeichnet, daß** die Anzahl der Mittel zum Zerkleinern mit großem Ausmaß (3 oder 13) zwischen 1 und 10 liegt, vorzugsweise zwischen 1 und 4.

**7.** Vorrichtung nach einem der Ansprüche 1 bis 6, **dadurch gekennzeichnet, daß** das Behältnis (12) röhrenförmig ist.

**8.** Vorrichtung nach einem der Ansprüche 1 bis 6, **dadurch gekennzeichnet, daß** das Behältnis (12) scheibenförmig ist.

**9.** Vorrichtung nach Anspruch 7 oder 8, **dadurch gekennzeichnet, daß** jegliches magnetisches Mittel zum Zerkleinern mit großem Ausmaß (3 oder 13) durch die Drehbewegung von zumindest einem Permanentmagneten (16) um das Behältnis (2 oder 12) herum bewegt wird.

**10.** Vorrichtung nach Anspruch 7 oder 8, **dadurch gekennzeichnet, daß** jegliches magnetisches Mittel zum Zerkleinern mit großem Ausmaß (3 oder 13) durch die Drehbewegung von zumindest einem Permanentmagneten (5 oder 15) unterhalb des Behältnisses (2 oder 12) bewegt wird.

**11.** Vorrichtung nach Anspruch 8 oder nach einem der Ansprüche 9 oder 10, **dadurch gekennzeichnet, daß** jegliches magnetisches Mittel zum Zerkleinern mit großem Ausmaß (3) mit einem im wesentlichen kreisförmigen Rollenweg (7) zusammenwirkt, der an der Peripherie des Behältnisses (2) angeordnet ist.

**12.** Verfahren zum Lysieren von zumindest einem Typ an Mikroorganismen einer biologischen Probe, um zumindest einen intrazellulären biologischen Bestandteil freizusetzen, bei dem in einem Behältnis zumindest ein Material in Form von Teilchen eingesetzt wird, das Mittel zum Zerkleinern bildet, die relativ hart und im wesentlichen inert gegenüber den biologischen Bestandteilen der Probe sind, **dadurch gekennzeichnet, daß** es folgende Schritte aufweist, nämlich

- Anordnen in dem Behältnis von zumindest einem magnetischen Mittel zum Zerkleinern mit großem Ausmaß, von zumindest einem nicht magnetischen Mittel zum Zerkleinern mit kleinem Ausmaß und einer biologischen Probe, die den zu lysierenden Mikroorganismus enthält, wobei die Mittel zum Zerkleinern mit großein Ausmaß ein Maß aufweisen, das größer ist als das der Mittel zum Zerkleinern mit kleinem Ausmaß, und wobei die Mittel zum Zerkleinern mit kleinem Ausmaß durch die Mittel zum Zerkleinern großen Ausmaßes bewegt werden,
- Plazieren der magnetischen Mittel zum Zerkleinern in ein mobiles Magnetfeld, um in der Mischung eine Wirbelbewegung zu erzeugen, und
- Anhalten der Einwirkung des Magnetfeldes auf die genannten magnetischen Mittel zum Zerkleinern.

**13.** Verfahren nach Anspruch 12, **dadurch gekennzeichnet, daß** das Magnetfeld im Bereich der biologischen Probe um das Behältnis drehbeweglich ist.

14. Verfahren nach Anspruch 12, **dadurch gekennzeichnet, daß** das Magnetfeld im Bereich der biologischen Probe unter dem Behältnis drehbeweglich ist.

15. Verfahren nach Anspruch 13 oder 14, **dadurch gekennzeichnet, daß** das drehbewegliche Magnetfeld den in der biologisehen Probe angeordneten magnetischen Mitteln zum Zerkleinern ein alternativ starkes und schwaches Feld auferlegt.

**Claims**

1. Device (1 or 11) for the lysis of at least one species of micro-organism, so as to release at least one intracellular biological component, which comprises a container (2 or 12) intended to receive a biological sample in liquid medium, which contains the micro-organism to be lysed, at least one material in the form of particles, which is relatively hard and substantially inert with respect to the biological compounds of the sample being present in this container, **characterized in that** the material in the form of particles comprises at least two types of grinding means of different sizes:

   - at least one large-sized magnetic means (3 or 13) which is slaved to the movement of a magnetic field, and
   - at least one small-sized means (4) which is set in motion by the large-sized grinding means (3 or 13).

2. Device according to Claim 1, **characterized in that** the material in the form of particles comprises, by way of large-sized grinding means (3 or 13), at least one large-diameter bead (3 or 13) and, by way of small-sized grinding means (4), at least one small-diameter bead (4).

3. Device according to either one of Claims 1 and 2, **characterized in that** the ratio between the sizes of the small-sized grinding means (4) and the size of the large-sized grinding means (3 or 13) is between 1/10 and 1/100, preferably between 1/30 and 1/60 and, more specifically, this ratio is 1/40.

4. Device according to any one of Claims 1 to 3, **characterized in that** the ratio between the size of the micro-organism to be lysed and the size of the small-sized grinding means (4) is between 1/10 and 1/100, preferably between 1/30 and 1/60, and more specifically is 1/50.

5. Device according to any one of Claims 1 to 4, **characterized in that** the ratio between the total volume of the small-sized grinding means (4) and the volume of the biological sample containing the micro-organism to be lysed is between 1/2 and 1/5 and is preferably 1/3.

6. Device according to any one of Claims 1 to 5, **characterized in that** the number of large-sized grinding means (3 or 13) is between 1 and 10 and preferably between 1 and 4.

7. Device according to any one of Claims 1 to 6, **characterized in that** the container (12) is of tubular shape.

8. Device according to any one of Claims 1 to 6, **characterized in that** the container (12) is of discoid shape.

9. Device according to Claim 7 or 8, **characterized in that** each large-sized magnetic grinding means (3 or 13) is moved by the rotary motion about the container (2 or 12) of at least one permanent magnet (16).

10. Device according to Claim 7 or 8, **characterized in that** each large-sized magnetic grinding means (3 or 13) is moved by the rotary motion under the container (2 or 12) of at least one permanent magnet (5 or 15).

11. Device according to Claim 8 and according to either one of Claims 9 and 10, **characterized in that** each large-sized magnetic grinding means (3) collaborates with a roughly circular runway (7) located at the periphery of the container (2).

12. Method for the lysis of-at least one species of micro-organism in a biological sample so as to release at least one intracellular biological component, which uses, in a container, at least one material in the form of particles, forming a grinding means which is relatively hard and substantially inert with respect to the biological compounds in the sample, **characterized in that** it consists in:

- placing at least one large-sized magnetic grinding means, at least one small-sized non-magnetic grinding means and a biological sample containing the micro-organism that is to be lysed in the container, the large-sized grinding means being larger in size than the small-sized grinding means, and the small-sized grinding means being set in motion by the large-sized grinding means,
- placing the magnetic grinding means in a moving magnetic field so as to generate a vortex motion in the mixture, and
- stopping the effect of the magnetic field on said magnetic grinding means.

13. Method according to Claim 12, **characterized in that** the magnetic field can rotate about the container at the height of the biological sample.

14. Method according to Claim 12, **characterized in that** the magnetic field can rotate under the container at the height of the biological sample.

15. Method according to either one of Claims 13 and 14, **characterized in that** the rotary magnetic field applies to the magnetic grinding means located in the biological sample a field which is alternately strong and weak.

EP 1 098 956 B1

Fig. 1

Détail B

Coupe A-A

Fig. 2

Détail B

Fig. 3

16

Fig. 4

F3

Fig. 5

E

F2

Fig. 6

F1